(19) Europäisches Patentamt
European Patent Office
Office européen des brevets

(11) Veröffentlichungsnummer: **0 036 519**
**B1**

# (12) EUROPÄISCHE PATENTSCHRIFT

(45) Veröffentlichungstag der Patentschrift: **02.03.83**

(51) Int. Cl.³: **C 07 D 233/58,** C 07 D 233/56, C 07 D 233/54

(21) Anmeldenummer: **81101534.6**

(22) Anmeldetag: **04.03.81**

## (54) Verfahren zur Herstellung von Imidazolen.

(30) Priorität: **13.03.80 DE 3009605**

(43) Veröffentlichungstag der Anmeldung: **30.09.81 Patentblatt 81/39**

(45) Bekanntmachung des Hinweises auf die Patenterteilung: **02.03.83 Patentblatt 83/9**

(84) Benannte Vertragsstaaten: **BE CH DE FR GB LI**

(56) Entgegenhaltungen:
**EP-A-0 000 208**
**FR-A-2 165 514**

(73) Patentinhaber: **BASF Aktiengesellschaft, Carl-Bosch-Strasse 38, D-6700 Ludwigshafen (DE)**

(72) Erfinder: **Dockner, Toni, Dr., Grossgasse 6, D-6701 Meckenheim (DE)**
Erfinder: **Gallei, Ewald, Dr., August-Bebel-Strasse 48, D-6806 Viernheim (DE)**
Erfinder: **Kempe, Uwe, Dr., Carl-Bosch-Strasse 44, D-6703 Limburgerhof (DE)**
Erfinder: **Krub, Herbert, Mussbacher Strasse 49, D-6700 Ludwigshafen (DE)**
Erfinder: **Magnussen, Peter, Dr., Professor-Dillinger-Weg 25, D-6702 Bad Duerkheim (DE)**
Erfinder: **Praetorius, Werner, Dr., Dirmsteiner Weg 47, D-6700 Ludwigshafen (DE)**
Erfinder: **Schwarz, Helmut, Dr., Weinbrennerstrasse 5, D-6700 Ludwigshafen (DE)**
Erfinder: **Romberg, Helmut, Dr., Amsterdamer Strasse 14, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 036 519 B1

BUNDESDRUCKEREI BERLIN

# Verfahren zur Herstellung von Imidazolen

Die Erfindung betrifft ein neues Verfahren zur Herstellung von Imidazolen durch Umsetzung von N,N'-Diformyl-1,2-diaminen bei Temperaturen von 350 bis 500°C in Gegenwart von Molybdänoxid und Kobaltoxid und/oder Nickeloxid als Katalysatoren.

Es ist aus der deutschen Offenlegungsschrift 1 952 991 bekannt, daß man Alkylendiamine mit Carbonylverbindungen in Gegenwart eines Kupfer- und/oder Chrom-Katalysators bei 320 bis 650°C zu Imidazolen umsetzen kann. Zusätzlich wird Wasserstoff der Reaktion zugeführt. Die Herstellung von in 2-Stellung unsubstituierten Imidazolen durch Umsetzung der entsprechenden Alkylendiamine mit Ameisensäure oder Formaldehyd gelingt auf diesem Wege jedoch nur in mäßigen Ausbeuten.

Die US-Patentschrift 2 847 417 beschreibt die Herstellung von Imidazolen aus Alkylendiaminen und Carbonsäuren an Trägerkatalysatoren der Platinmetalle. Solche Katalysatoren sind teuer und werden durch bestimmte Stoffe wie Schwefel, Schwermetalle oder Halogene leicht vergiftet. Edelmetallkatalysatoren verlangen aufwendige Herstellungsverfahren, um die gleichmäßige Wirksamkeit des Katalysators zu gewährleisten, sowie sehr reine Ausgangsstoffe, um die Wirkung des Katalysators nicht zu beeinträchtigen. Zusätzlich wird Wasserstoff zugesetzt, um die Bildung teeriger Polymere und Ablagerungen auf dem Katalysator zu vermeiden und die Wirksamkeit des Katalysators so möglichst lange zu erhalten.

Nach den Angaben der deutschen Patentschrift 1 231 249 läßt sich Imidazol durch Umsetzung von N,N'-Diformyläthylendiamin in der Gasphase an Zinkoxidkatalysatoren bei Temperaturen zwischen 250 und 800°C herstellen. Die Imidazolausbeute beträgt bei diesem Verfahren zwischen 42% und 80% der Theorie. Alle Beispiele zeigen, daß zusätzlich Stickstoff als Schlepp- oder Verdünnungsmittel zugeführt wird, wobei die Konzentration von 1 bis 999 Mol Stickstoff je Mol Ausgangsstoff beträgt. Nach den Angaben der Patentschrift sind die Ausbeuten bei Verwendung von Zinkoxid als dem einzigen Katalysator unbefriedigend. Wie die Beschreibung (Spalte 2, Zeilen 29 bis 36) zeigt, werden die Katalysatoren in einfacher Weise hergestellt und weisen keine besonderen Strukturmerkmale, lediglich eine Korngröße von 0,2 bis 0,4 mm auf. Im Gegensatz zu dem in der US-Patentschrift 2 847 417 beschriebenen Verfahren, dessen Ausbeuten in der Beschreibung als sehr gering angegeben werden, wird kein Wasserstoff verwendet; in allen Beispielen kommt Stickstoff in einer Menge von 45 bis 60 Mol je Mol Ausgangsstoff zur Anwendung. Die beste Ausbeute an Imidazol von 80% (Beispiel 2) wird mit einem Katalysator von 78 Gewichtsprozent Zinkoxid, 7 Gewichtsprozent Aluminiumoxid, 5 Gewichtsprozent Calciumoxid, 5 Gewichtsprozent Kaliumsulfat, 2 Gewichtsprozent Magnesiumoxid, 1 Gewichtsprozent Chrom-III-oxid, 2 Gewichtsprozent Eisen-III-oxid, Natriumoxid und Kaliumoxid bei 500°C in Gegenwart von Stickstoff erhalten.

Die nachgängige deutsche Offenlegungsschrift 2 729 017 verwendet im Gegensatz zu dem Verfahren der deutschen Patentschrift 1 231 249 keine N,N'-Diformyl-1,2-diamine, sondern setzt 1,2-Diamine mit Ameisensäure an Zinkoxid bzw. an Zinkoxid und Aluminiumoxid, bevorzugt von bestimmter Struktur, bei 300 bis 600°C um. Es wird ausdrücklich festgestellt, daß bei diesem Katalysator ein Zusatz von Wasserstoff nicht zweckmäßig und auch nicht notwendig ist, da eine Bildung teeriger Polymere, Ablagerungen auf dem Katalysator und ein rascher Rückgang der Katalysatoraktivität nicht beobachtet werden. Ein Vergleich der Herstellungsweise von Imidazol (Beispiel 2) mit der besten Verfahrensweise der deutschen Offenlegungsschrift 1 231 249 (Beispiel 2) zeigt, daß der besonders strukturierte Zinkoxid/Aluminiumoxid-Katalysator auch in Gegenwart von Stickstoff und bei einer Temperatur von 550°C eine wesentlich schlechtere (69,2%) Ausbeute als der aus 9 Komponenten und beliebig strukturiertem Zinkoxid gebildete Katalysator im Beispiel 2 der Patentschrift liefert.

Die französische Patentschrift 1 266 702 beschreibt die Umsetzung eines Gemischs von einem Alkylendiamin und Formaldehyd bei vorteilhaft 315 bis 482°C an einem Edelmetallkatalysator auf Träger; in allen Beispielen wird ein Platinkatalysator auf Aluminiumoxid verwendet. Die Ausbeuten sind unbefriedigend. Es wird erwähnt, daß zwar Kobaltmolybdat auf einem Träger als Katalysator verwendet werden kann,aber darauf aufmerksam gemacht (Seite 2, letzter Absatz), daß nicht notwendigerweise gleich gute Ergebnisse erhalten werden. Die Patentschrift erläutert, daß nur die echte Verbindung Kobaltmolybdat und keine Gemische von Kobaltoxid und Molybdänoxid verwendet werden können. Als Träger wird Aluminiumoxid angegeben.

Es wurde nun gefunden, daß man Imidazole der Formel

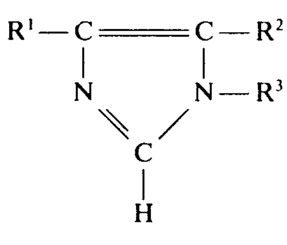

(I)

worin $R^1$, $R^2$ und $R^3$ gleich oder verschieden sein können und jeweils einen aliphatischen, cycloaliphatischen, araliphatischen oder aromatischen Rest oder ein Wasserstoffatom bedeuten, durch Umsetzung von N,N'-Diformyl-1,2-diaminen bei erhöhter Temperatur in Gegenwart von Metallkatalysatoren vorteilhaft erhält, wenn man N,N'-Diformyl-1,2-diamine der Formel

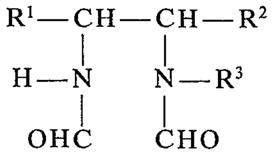

(II)

worin $R^1$, $R^2$ und $R^3$ die vorgenannte Bedeutung besitzen, bei einer Temperatur von 350 bis 500°C in Gegenwart von

a) Molybdänoxid und
b) Nickeloxid und/oder Kobaltoxid als Katalysatoren umsetzt.

Weiterhin wurde gefunden, daß man das Verfahren vorteilhaft ausführt, wenn man die Umsetzung in einem Wirbelbett durchgeführt, wobei ein Teil des Katalysators aus dem Reaktionsraum entnommen und der Regenerierung zugeführt wird und regenerierter Katalysator in den Reaktionsraum eingegeben wird.

Die Umsetzung kann für den Fall der Verwendung von N,N'-Diformyl-1,2-diaminopropan durch die folgenden Formeln wiedergegeben werden:

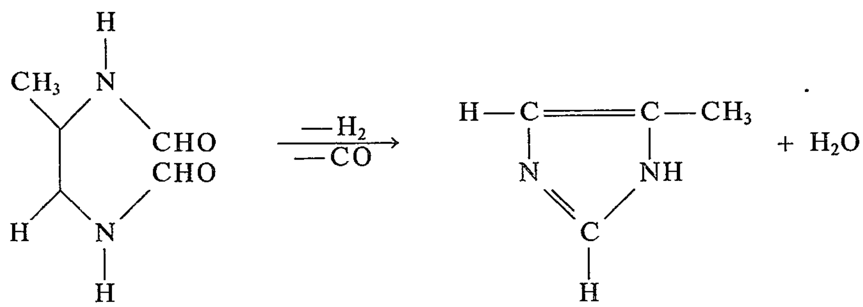


Im Vergleich zu den bekannten Verfahren liefert das Verfahren nach der Erfindung auf einfacherem und wirtschaftlicherem Wege Imidazole in besserer Raum-Zeit-Ausbeute. Die Bildung teeriger Polymere, die zu einem raschen Rückgang der Katalysatoraktivität führen, wird nicht beobachtet. Die erfindungsgemäßen Katalysatoren sind wirtschaftlich und leicht regenerierbar. Insbesondere liefert das Verfahren im Hinblick auf die Verfahren mit Zinkoxid oder Edelmetalle enthaltenden Katalysatoren weniger Mono-, Di- oder Trialkylimidazole als Nebenprodukte. Bei den bekannten Verfahren ist der Endstoff insbesondere mit ein- und zweifach alkylierten Imidazolen, z. B. 1-, 4-, 2-Alkylimidazol, 2,4-Dialkylimidazol, 2,5-Dialkylimidazol, verunreinigt, die nur schwer abtrennbar sind. Entsprechende zusätzliche Anlagen zur fraktionierten Destillation werden eingespart. Die erfindungsgemäßen Katalysatoren zeigen höhere Aktivität und Lebensdauer. Alle diese vorteilhaften Eigenschaften sind im Hinblick auf den Stand der Technik überraschend. So hätte man im Hinblick auf die deutsche Patentschrift 1 231 249 nicht vermuten können, daß gerade der erfindungsgemäße Katalysator, obwohl er nicht die 9 Komponenten des in der Patentschrift beschriebenen Katalysators enthält und kein Zinkoxid besonderer Struktur aufweist, höhere Raum-Zeit-Ausbeuten an Endstoff liefert. Hinzu kommt, daß man im Hinblick auf die Lehre der deutschen Offenlegungsschrift 2 729 017 die Verwendung der freien Amine mit Ameisensäure und nicht die der N,N'-Diformylverbindungen als vorteilhafte Arbeitsweise betrachtet hätte. Auch war mit Bezug auf die französische Patentschrift 1 266 702 überraschend, daß die erfindungsgemäßen Ausgangsstoffe anstelle von Gemischen von Alkylendiaminen und Formaldehyd und noch dazu unter Verwendung der erfindungsgemäßen Katalysatoren diese vorteilhaften Ergebnisse liefern.

Die N,N'-Diformyl-1,2-diamine II können durch Reaktion der entsprechenden Diamine mit Ameisensäureestern, Formamid oder Ameisensäure, z. B. im Molverhältnis 1 : 2 bis 4, vorteilhaft 1 : 2 bis 2,5, vorzugsweise bei erhöhter Temperatur und Druck, zweckmäßig 50 bis 130°C und 2 bis 10 bar, hergestellt werden. Bevorzugte Ausgangsstoffe II und dementsprechend bevorzugte Endstoffe I sind solche, in deren Formeln $R^1$, $R^2$ und $R^3$ gleich oder verschieden sein können und jeweils einen Alkylrest mit 1 bis 18 Kohlenstoffatomen, insbesondere mit 1 bis 8 Kohlenstoffatomen, einen Alkenylrest mit mehreren oder insbesondere einer Doppelbindung und mit 2 bis 18, vorzugsweise 3 bis 18, insbesondere 4 bis 8 Kohlenstoffatomen, einen Cyclohexylrest, einen Aralkylrest oder Alkylarylrest mit 7 bis 12 Kohlenstoffatomen, einen Phenylrest oder ein Wasserstoffatom bedeuten. Die vorgenannten Reste können noch durch unter den Reaktionsbedingungen inerte Gruppen, z. B. Alkylgruppen oder

Alkoxygruppen mit jeweils 1 bis 4 Kohlenstoffatomen, substituiert sein.

Als Ausgangsstoffe II kommen z. B. in Frage: Die N,N'-Diformylverbindungen von Äthylendiamin, 1,2-Propylendiamin, 1,2-Butylendiamin, 1,2-Pentylendiamin, 1,2-n-Hexylendiamin, 1,2-n-Heptylendiamin, 1,2-n-Octylendiamin, 1,2-n-Nonylendiamin, 1,2-n-Decylendiamin, 1,2-n-Octadecylendiamin; 2,3-Butylendiamin, 2,3-Pentylendiamin, 2,3-Hexylendiamin, 2,3-Heptylendiamin, 2,3-Octylendiamin, 2,3-Nonylendiamin, 2,3-Decylendiamin, 3,4-Hexylendiamin, 3,4-Heptylendiamin, 3,4-Octylendiamin, 3,4-Nonylendiamin, 3,4-Decylendiamin, 4,5-Octylendiamin, 4,5-Nonylendiamin, 4,5-Decylendiamin, 5,6-Decylendiamin; durch die Benzyl-, Cyclohexyl- und/oder Phenylgruppe in 1-Stellung einfach oder in 1- und 2-Stellung gleichzeitig substituierten Äthylendiaminen; durch vorgenannte Alkylgruppen in 1-Stellung und durch die Benzyl-, Cyclohexyl- oder Phenylgruppe in 2-Stellung substituierten Äthylendiamine; homologen, durch die Methyl-, Äthyl-, Allyl-, Crotyl-, Propyl-, Isopropyl-, Butyl-, Isobutyl-, Pentyl-, sek.-Butyl-, tert.-Butyl-, Hexyl-, Heptyl-, Octyl-, Nonyl-, Decyl-, Octadecyl-, Cyclohexyl-, Cyclopentyl-, Benzyl-, Tolyl-, Xylyl-, Naphthyl-, Methoxyphenyl-, Äthoxyphenyl-, Äthylphenyl-, Dimethoxyphenylgruppe mono-N-substituierten 1,2-Diaminen.

Die Umsetzung wird bei einer Temperatur von 350 bis 500°C, zweckmäßig von 400 bis 480°C, drucklos oder unter Druck, diskontinuierlich oder bevorzugt kontinuierlich durchgeführt. In der Regel dient das Reaktionsgemisch auch als Lösungsmedium.

Als Katalysator werden Nickeloxid oder Kobaltoxid allein oder ein Gemisch von Nickeloxid und Kobaltoxid, stets zusammen mit Molybdänoxid, zweckmäßig in einem Verhältnis von 0,1 bis 50, vorzugsweise 0,2 bis 2 Mol Nickeloxid und/oder Kobaltoxid zu 1 Mol Molybdänoxid, verwendet. Vorteilhaft ist ein Verhältnis von 0,1 bis 10, vorzugsweise 0,2 bis 3 Mol Molybdänoxid je Mol Ausgangsstoff II. Der Katalysator kann gegebenenfalls noch in katalytischen Mengen, zweckmäßig 0,1 bis 10 Gewichtsprozent Kalium- und/oder Natriumsulfat, 0,1 bis 5 Gewichtsprozent Magnesiumoxid und/oder Eisen-III-oxid und/oder 0,1 bis 3 Gewichtsprozent Phosphorsäure enthalten. Der Katalysator kann trägerfrei sein oder auf einem Träger, vorteilhaft in einer Menge von 1 bis 18 Gewichtsprozent Katalysator, bezogen auf den Träger, aufgebracht sein. Als Träger kommen zweckmäßig Aluminiumverbindungen, bevorzugt Aluminiumoxide, und Kieselsäureverbindungen in Frage. Als Aluminiumoxid kommen z. B. $\alpha$-, $\delta$- und $\gamma$-Aluminiumoxid oder anstelle von Aluminiumoxid auch dieses Oxid enthaltende Stoffe oder Stoffgemische in Betracht, z. B. Aluminiumsilikat, Magnesiumaluminiumsilikathydrat, Dimagnesiumaluminiumsilikathydrat, Natriumaluminiumsilikat, Calciumaluminiumsilikat, Fullererde, Tone, Bleicherden wie Bentonit, Bauxit, Bimsstein, Andalusit, Kaolin, Allophane, Zeolithe, Mullit, Korund, $\gamma$-Tonerde, Hydrargillit, Böhmit. Als Kieselsäureverbindungen kommen in Frage: Silikate, z. B. Montmorillonit, Floridaerde, Quarz, Asbest; gefällte Kieselsäure, Kieselgel, Kieselgur. Weitere Träger sind Titandioxid, Zirkondioxid, Zinndioxid, Aktivkohle; Erdalkalisulfate oder Erdalkaliphosphate, z. B. die Calcium- oder Bariumsalze; oder entsprechende Gemische vorgenannter Trägermaterialien. Die Herstellung der Trägerkatalysatoren wird nach den üblichen Verfahren, z. B. durch Auftragen einer Kobalt- und/oder Nikkelverbindung und einer Molybdänverbindung auf den Träger, Trocknen und Calcinieren, beispielsweise zwischen 400 und 1200°C in reduzierender, oxidierender oder inerter Atmosphäre, durchgeführt. Der Träger kann auch in seiner gewünschten geometrischen Form mit einer Lösung einer Molybdänverbindung allein bzw. der Nickel-, Kobalt- und Molybdänverbindung, z. B. einer wäßrigen Lösung von Nickelsulfat und Molybdänsulfat, getränkt und getrocknet werden. Ebenfalls kann man das Trägermaterial mit vorgenannten Metallverbindungen und Wasser verkneten, in die gewünschte Form bringen, trocknen und bei einer Temperatur von 400 bis 1200°C calcinieren. In allen diesen Fällen kommen als Metallverbindungen solche, die unter den Reaktionsbedingungen die entsprechenden Metalloxide bilden, in Betracht, z. B. die Sulfate, Nitrate, Chloride, Bromide, Carbonate, Dicarbonate, Formiate, Acetate von Nickel, Kobalt und Molybdän.

Die Teilchengröße der Katalysatoren beträgt vorzugsweise von 0,005 bis 7, insbesondere 2 bis 4 Millimeter. Die Form kann beliebig, z. B. in Pillen-, Zylinder- oder Strangform, kugelförmig oder körnig, gewählt werden. Der Katalysator besitzt zweckmäßig Porenvolumina von 0,1 bis 0,6 Milliliter je Gramm, vorzugsweise von 0,2 bis 0,4 Milliliter je Gramm, spezifische Oberfläche von 10 bis 200 Quadratmeter je Gramm, vorzugsweise von 50 bis 100 Quadratmeter je Gramm, und Schüttgewichte von 0,4 bis 0,9, vorzugsweise von 0,5 bis 0,8 Gramm je Milliliter.

Vorzugsweise werden die Katalysatoren auf dem Träger in Splitt- oder Kugelform in der Wirbelschicht eingesetzt, wobei zweckmäßig Katalysatorteilchen mit Korngrößen von 0,005 bis 3 mm, insbesondere von 0,1 bis 1 mm, bevorzugt 0,2 bis 0,4 mm, verwendet werden. Die Schichthöhe des Katalysatorbettes im Wirbelzustand beträgt vorteilhaft 30 bis 2000 Millimeter oder wird zweckmäßig so gewählt, daß sich Verweilzeiten der Ausgangsstoffe II in der Katalysatorschicht von 0,01 bis 20, vorzugsweise von 5 bis 10 Sekunden ergeben. Bezüglich der Herstellung der Katalysatoren wird auf Houben—Weyl, Methoden der Organischen Chemie, Band 4/2, Seiten 142 ff. und Ullmanns Encyklopädie der technischen Chemie, Band 9, Seiten 271 ff., verwiesen.

Die Umsetzung wird vorteilhaft in Gegenwart von Inertgasen durchgeführt, zweckmäßig von Stickstoff, CO, $CO_2$ oder Wasserstoff. Man verwendet zweckmäßig Mengen von 1 bis 200, insbesondere 10 bis 50 Mol Stickstoff oder $CO/H_2$-Gemische in Mengen von 1 bis 100, vorteilhaft 2 bis 50, insbesondere 3 bis 18 Mol Kohlenmonoxid und 1 bis 100, vorteilhaft 3 bis 50, insbesondere 6 bis 20 Mol Wasserstoff je Mol Ausgangsstoff II. Die Gemische können noch weitere gasförmige bzw. dampfförmige

Komponenten enthalten. Bevorzugt wird das Abgas der erfindungsgemäßen Reaktion zurückgeführt (Kreisgas) und als Gasgemisch von $CO/H_2$ verwendet. Vorteilhaft ist ein Kreisgas von 30 bis 90, insbesondere 50 bis 75 Gewichtsprozent CO, von 3 bis 15, insbesondere 5 bis 7 Gewichtsprozent Wasserstoff, das im allgemeinen noch von 0 bis 50, insbesondere 10 bis 40 Gewichtsprozent $CO_2$, 0 bis 30, insbesondere von 0 bis 10 Gewichtsprozent Stickstoff, 0 bis 10, insbesondere 0,5 bis 5 Gewichtsprozent Ammoniak, 0 bis 15, insbesondere 0,5 bis 10 Gewichtsprozent dem Ausgangsstoff II zugrundeliegendes Diamin, 0 bis 12, insbesondere 0,1 bis 6 Gewichtsprozent Methan enthält.

Die Reaktion kann wie folgt durchgeführt werden: Der feste, verflüssigte oder zweckmäßig dampfförmige Ausgangsstoff II wird im Gemisch mit Inertgas, zweckmäßig in Form des Abgases der Reaktion als Kreisgas, bei der Reaktionstemperatur über den Katalysator bzw. Katalysator auf dem Träger in einem Festbett geleitet. Das aus dem Reaktor dampfförmig austretende Reaktionsgemisch wird gegebenenfalls in einem Zyklon entstaubt und in einer gekühlten Vorlage kondensiert. Durch fraktionierte Destillation wird dann zweckmäig der Endstoff abgetrennt. Der Endstoff kann auch durch Umkristallisation oder Umfällung aus geeigneten Lösungsmitteln, z. B. mit Toluol, Dimethylformamid, oder verdünnten Säuren, z. B. mit Ameisensäure, isoliert werden.

In einer bevorzugten Ausführungsform des Verfahrens wird der Ausgangsstoff II in einer Wirbelschicht bei der Reaktionstemperatur umgesetzt. Der Katalysator bzw. Katalysator auf Träger kann durch das Ausgangsgemisch oder das Inertgas allein, zweckmäßig in Gestalt des vorgenannten Kreisgases, als Wirbelschichtgas bei Normaldruck oder vermindertem oder erhöhtem Druck in einer Wirbelschicht gehalten werden. Entsprechend kann die Gesamtmenge oder eine Teilmenge an Ausgangsstoff II getrennt von dem Wirbelschichtgas in den Wirbelschichtreaktor eingeleitet werden. Das Diamin II wird zweckmäßig in einem beheizten Vorratsgefäß flüssig gehalten und in einen Verdampfer dosiert, der dem Wirbelschichtreaktor vorgeschaltet ist. Gleichzeitig leitet man vorteilhaft einen schwachen Kreisgasstrom, zweckmäßig von 5000 bis 50 000 Volumenteilen des vorgenannten Kreisgases je Stunde, durch den Verdampfer. Der verdampfte Ausgangsstoff wird zusammen mit dem Kreisgasstrom durch das Katalysatorbett geleitet. Ebenfalls kann man aber auch den Ausgangsstoff II in fester Form, vorteilhaft in einer Korngröße zwischen 0,1 bis 3 Millimeter, oder in flüssiger Form direkt in den Wirbelschichtreaktor eindosieren, wobei die Zudosierung des Ausgangsstoffs II getrennt von dem Wirbelgas oder zusammen mit ihm erfolgen kann. Man kann das Verfahren nach der Erfindung in einem einfachen oder unterteilten, offenen oder geschlossenen Wirbelschichtsystem mit und ohne Fließstaubzirkulation durchführen. Bezüglich Reaktoren, Durchführung, Verfahrensvarianten und Reaktionsbedingungen des Wirbelschichtverfahrens wird auf Ullmanns Encyklopädie der technischen Chemie, Band 1, Seiten 916 bis 933, verwiesen. Die Aufarbeitung des Reaktionsgemisches erfolgt in vorgenannter Weise.

In einer vorteilhaften Ausführungsform führt man nach der Reaktion das dampfförmige Reaktionsgemisch in eine Trennkolonne, zieht aus dem Sumpf den Endstoff ab und führt über den Kopf der Kolonne das restliche Gemisch in eine Vorlage, wo die kondensierbaren Anteile, im wesentlichen Wasser und das dem Ausgangsstoff I zugrundeliegende Diamin, kondensiert werden. Ein Teil des Abgases, zweckmäßig ein Anteil, der die vorgenannten Mengen an CO und $H_2$ enthält, wird in eine Waschkolonne geführt, anschließend getrocknet und als Kreisgas zurückgeführt.

In einer bevorzugten Ausführungsform wird im Wirbelbett umgesetzt und drucklos oder unter Druck, diskontinuierlich oder kontinuierlich gleichzeitig dem Wirbelreaktor ein Katalysatoranteil, zweckmäßig von 1 bis 100, vorzugsweise 20 bis 50 Gewichtsprozent Katalysator je 24 Stunden Betriebsdauer des Wirbelbetts, bezogen auf die Gesamtmenge des Katalysators im Wirbelbett, entzogen und eine entsprechende, regenerierte Katalysatormenge zugeführt. Die Betriebsdauer des Wirbelbetts kann die Dauer eines kontinuierlichen Betriebs oder die Dauer des Betriebs mehrerer diskontinuierlicher Umsetzungen mit demselben Katalysator bedeuten. In einem kontinuierlichen Betrieb, insbesondere im Wirbelreaktor, wird in der Regel das vorgenannte Verhältnis von Nickeloxid und/oder Kobaltoxid und von Molybdänoxid je Stunde und Mol Ausgangsstoff II verwendet. Zuführung und Entnahme des Katalysators kann mit den üblichen Apparaturen des Feststofftransports, z. B. Förderpumpen, Transportschnecken, pneumatische Förderung erfolgen.

Der so entnommene Katalysator kann kontinuierlich, zweckmäßig im Kreislauf von der Entnahme über die Regenerierung zur Zuführung in das Wirbelbett, oder diskontinuierlich regeneriert werden. Die Regenerierung (Reaktivierung) des Katalysators erfolgt zweckmäßig in einem vorgenannten Wirbelreaktor. Die Schichthöhe im Regenerierungswirbelreaktor beträgt zweckmäßig 30 bis 2000 Millimeter, die Verweilzeit des Katalysators im Regenerierungswirbelreaktor 1 bis 100, vorzugsweise 3 bis 50 Stunden. Als Wirbelschichtgas im Regenerierungswirbelreaktor (Regeneriergas) können die vorgenannten Inertgase, Gasgemische und Kreisgas, gegebenenfalls unter Zusatz von Wasserdampf, und Luft bzw. Sauerstoff verwendet werden. Die Regenerierung wird in der Regel bei einer Temperatur von 300 bis 600, vorzugsweise 400 bis 500°C, drucklos oder unter Druck, zweckmäßig mit 0 bis 99,9, vorzugsweise 80 bis 99 Volumenteilen Inertgas bzw. Inertgasgemisch je Stunde und Kilogramm Katalysator, mit Luft oder Sauerstoff, vorzugsweise in einer Menge von 0,01 bis 1, insbesondere 0,05 bis 0,5 Mol Sauerstoff je Stunde und Mol Nickeloxid, Kobaltoxid und Molybdänoxid, durchgeführt. Der Sauerstoffgehalt im Regeneriergas beträgt im allgemeinen 0,1 bis 100 Gewichtsprozent, zweckmäßigerweise 0,3 bis 20 Gewichtsprozent. Gegebenenfalls kann der Katalysator nach der Entnahme in einem Zyklon vom Katalysatorstaub befreit werden bzw. zusätzlicher Katalysator zum Ersatz von Katalysatorverlusten der erfindungsgemäßen Reaktion wieder zugeführt werden. Diese Ausführungsform der kontinuierlichen Regenerierung des Katalysators vermeidet Abstellung der Anlage und Unterbrechungen der Reaktion zur Durchführung der Regenerierung der gesamten Reaktorfüllung. Eine kontinuierliche Produktion wird gewährleistet. Gleichzeitig kann gebildeter Katalysatorstaub einfach aus dem Reaktionssystem entfernt werden. Ein Nachlassen der Katalysatoraktivität durch die Regenerierung wird nicht beobachtet. Die Ausbeuten werden nicht beeinträchtigt.

Die nach dem Verfahren der Erfindung herstellbaren Imidazole I sind wertvolle Ausgangsstoffe für die Herstellung von Farbstoffen, Pflanzenschutzmitteln, Textilhilfsmitteln, Katalysatoren für Polyurethane und Epoxidharze, oberflächenaktiven Mitteln und Pharmazeutika, z. B. den entsprechenden Nitroimidazolen. Imidazole I werden als Katalysatoren für Polymerisationsreaktionen und Aldolkondensationen verwendet. Bezüglich der Verwendung wird auf die vorgenannten Veröffentlichungen und Ullmanns Encyklopädie der technischen Chemie (3. Auflage), Band 8, Seite 499, und (4. Auflage), Band 13, Seiten 173 bis 175, verwiesen.

Die in den folgenden Beispielen aufgeführten Teile bedeuten Gewichtsteile. Die Gewichtsteile verhalten sich zu den Volumenteilen wie Kilogramm zu Liter.

## Beispiel 1

### a) Herstellung des Ausgangsstoffs II

180 Teile 1,2-Diaminoäthan werden unter Rühren und Kühlung mit 400 Teilen Ameisensäuremethylester bei 25 bis 30°C gemischt. Nach dem Abkühlen des Reaktionsgemisches wird der Niederschlag abgesaugt und getrocknet. Man erhält 343 Teile (98,5%) der Theorie) N,N'-Diformyl-diaminoäthan vom Fp 111°C.

### b) Reaktion

80 Teile N,N'-Diformyl-diaminoäthan werden pro Stunde aus einem Vorratsgefäß zusammen mit 550 000 Volumenteilen pro Stunde Stickstoff durch einen auf 400°C erhitzten Wirbelreaktor geleitet. Der Wirbelreaktor ist ein vertikales, elektrisch beheiztes Quarzrohr, das nach unten mit einer Quarzfritte abgeschlossen ist. Der feste Ausgangsstoff II wird seitlich in den Reaktor eindosiert. Das Quarzrohr ist mit 1000 Teilen eines Katalysators aus 6 Teilen NiO und 14 Teilen $MoO_3$ auf 80 Teilen Aluminiumsilikat (Korngröße 0,1 bis 0,3 mm) zur Hälfte gefüllt. Die den Reaktor verlassenden Dämpfe werden kondensiert und fraktioniert destilliert. Man erhält stündlich 30,8 Teile Imidazol (65,7% der Theorie, bezogen auf eingesetzten Ausgangsstoffe II) vom Fp 89°C. Der Umsatz beträgt 99,3 Prozent. Die Ausbeute bleibt auch noch nach 250 Stunden Betrieb konstant.

## Beispiel 2

a) 120 Teile 1,2-Diaminopropan werden pro Stunde bei 100°C und 4 bar Druck in einen Schlaufenreaktor geleitet. Gleichzeitig werden 225 Teile Ameisensäuremethylester pro Stunde zugeführt. Die Verweilzeit im Reaktor beträgt 5 Minuten. Das Reaktionsgemisch wird kontinuierlich destillativ getrennt, wobei überschüssiger Ameisensäuremethylester zurückgeführt wird. Man erhält 207 Teile (98,2% der Theorie) N,N'-Diformyl-1,2-diaminopropan vom Fp 64°C.

b) Stündlich werden 50 Teile geschmolzenes N,N'-Diformyl-1,2-diaminopropan bei 300°C verdampft und die Dämpfe zusammen mit 310 000 Volumenteilen pro Stunde Stickstoff bei 420°C durch einen elektrisch beheizten Festbettreaktor geleitet. Der Reaktor sitzt senkrecht auf dem Verdampfer und ist mit 5 mm langen und 2 mm dicken zylindrischen Füllkörpern (500 Teilen), bestehend aus 4 Teilen CoO und 15 Teilen $MoO_3$ auf 81 Teilen $\gamma$-Aluminiumoxid, gefüllt. Nach der Aufarbeitung analog Beispiel 1 erhält man stündlich 19 Teile (60,2% Ausbeute, bezogen auf eingesetzten Ausgangsstoff II) 4-Methylimidazol vom Fp 50°C. Der Umsatz beträgt 98,5%, bezogen auf Ausgangsstoff II. Die Ausbeute ist auch nach 200 Stunden Betrieb noch konstant.

## Beispiel 3

100 Teile flüssiges 2,3-Diamino-N,N'-diformylbutan werden stündlich zusammen mit 200 000 Teilen Kreisgas bei 435°C in einen Wirbelreaktor geleitet. Zusätzlich werden 350 000 Teile Kreisgas zugeführt. Das Kreisgas enthält 61 Gewichtsprozent CO, 6 Gewichtsprozent $H_2$, 6 Gewichtsprozent $NH_3$, 5 Gewichtsprozent Methan, 4 Gewichtsprozent Äthan und Äthylen und 23 Gewichtsprozent $CO_2$. Der Reaktor ist mit 2000 Teilen eines Wirbelkatalysators (3 Teile NiO, 15 Teile $MoO_3$, 75 Teile Aluminium-

oxid, 0,5 Teile $Na_2O$, 5,8 Teile $P_2O_5$) gefüllt. Die Korngröße des Katalysators beträgt 0,1 bis 0,3 mm. Das den Reaktor verlassende Reaktionsgemisch wird kondensiert und fraktioniert destilliert. Man erhält stündlich 39,1 Teile (57,7% der Theorie) 4,5-Dimethylimidazol vom Fp 112°C. Das die Destillationskolonne verlassende Kreisgas wird mit Wasser in einer Waschkolonne gereinigt, auf 5°C gekühlt und zurückgeführt.

Beispiel 4

50 Teile flüssiges N,N'-Diformyl-1,2-ethylendiamin werden stündlich dem in Beispiel 1 beschriebenen Wirbelreaktor zugeführt. Als Wirbelgas dienen 200 000 Teile eines äquimolaren Gemisches aus Wasserstoff und Kohlenstoffmonoxid. Der Wirbelreaktor ist mit einem Katalysator der Korngröße 0,1 bis 0,3 mm (3 Teile NiO, 15 Teile $MoO_3$ und 4 Teile Phosphorsäure auf 78 Teile $\gamma$-$Al_2O_3$) gefüllt. Die Umsetzung und Aufarbeitung erfolgt analog Beispiel 1. Man erhält stündlich 20,4 Teile (69,7% der Theorie) Imidazol vom Fp 89°C. Über 100 Stunden Betriebsdauer wird dieselbe Ausbeute erhalten.

Beispiel 5

Entsprechend Beispiel 4 werden 40 Teile pro Stunden N,N'-Diformyl-1,2-ethylendiamin (flüssig) zusammen mit 100 000 Teilen Kreisgas in einen Wirbelreakor geleitet, dem zusätzlich 178 000 Teile Kreisgas zugeführt werden. Der Reaktor ist mit 800 Teilen des in Beispiel 3 beschriebenen Katalysators der Korngröße 0,1 bis 0,3 mm gefüllt. Man erhitzt während 250 Stunden von 390°C Wirbelreaktortemperatur auf 470°C. Man erhält stündlich 17,2 Teile (73,3% der Theorie) Imidazol vom Fp 89°C. Über 250 Stunden wird dieselbe Ausbeute erhalten.

Beispiel 6

80 Teile N,N'-Diformyl-1,2-ethylendiamin werden stündlich bei 380°C als Feststoff zusammen mit 200 000 Teilen Stickstoff in einen ersten Wirbelreaktor geleitet. Zusätzlich werden 350 000 Teile Kreisgas stündlich zugeführt. Das Kreisgas enthält 78,5 Gewichtsprozent CO, 6,6 Gewichtsprozent $H_2$, 0,9 Gewichtsprozent $NH_3$, 3,8 Gewichtsprozent Methan, 0,3 Gewichtsprozent Äthan und Äthylen und 9,9 Gewichtsprozent $CO_2$. Der Reaktor ist mit 2000 Teilen eines Wirbelkatalysators (6 Teile NiO; 30 Teile $MoO_3$; 8 Teile Phosphorsäure; 1956 Teile $\gamma$-Aluminiumoxid) gefüllt.
Die Höhe der Katalysatorzone beträgt im Wirbelzustand 600 mm, die Verweilzeit in der Katalysatorzone im Wirbelzustand beträgt 6 Sekunden. Durch einen seitlich angebrachten Entnahmestutzen werden kontinuierlich stündlich 50 Teile des Katalysators ausgeschleust. Der ausgeschleuste Katalysator wird diskontinuierlich in einem zweiten (Regenerierungs-)Wirbelreaktor, der jeweils mit 5000 Teilen des gebrauchten Kontaktes bis zur Hälfte gefüllt ist, durch Zufuhr von stündlich 250 000 Teilen Stickstoff, 50 000 Teilen Wasserdampf und 2000 Teilen $O_2$ bei 440°C innerhalb von 24 Stunden regeneriert. 50 Teile des so regenerierten Katalysators werden stündlich zurückgeführt und zusammen mit dem Ausgangsstoff II seitlich in den ersten Wirbelreaktor eindosiert. Die den ersten Wirbelreaktor verlassenden Dämpfe werden in einem Zyklon entstaubt, kondensiert und fraktioniert destilliert. Ein Teil des verbleibenden Gasgemisches wird gewaschen, getrocknet und als Kreisgas zurückgeführt. Überschüssiges Kreisgas wird ausgeschleust. Man erhält stündlich 36,7 Teile Imidazol (78,3% der Theorie, bezogen auf eingesetzten Ausgangsstoff II) vom Fp 88°C. Der Umsatz beträgt 99,5%. Eine Ausbeuteverschlechterung ist auch nach 400 Stunden noch nicht festzustellen.

Beispiel 7

Bei der Umsetzung und Katalysatorregenerierung nach Beispiel 6 werden stündlich 100 Teile N,N'-Diformyl-2-phenyl-1,2-ethylendiamin zusammen mit 150 000 Teilen Stickstoff flüssig zugeführt, wobei weitere 400 000 Teile Stickstoff als Wirbelgas dienen. Man erhält 62 Teile (82,1% der Theorie) 4(5)-Phenylimidazol vom Schmelzpunkt Fp 127 bis 129°C. Der Umsatz beträgt 99,5%. Eine Ausbeuteverschlechterung ist auch nach 400 Stunden noch nicht festzustellen.

Beispiel 8

Die Umsetzung wird analog Beispiel 3 durchgeführt. Über einen separaten seitlichen Stutzen werden dem Wirbelreaktor kontinuierlich stündlich 100 Teile regenerierten Katalysators zugeführt. Über einen Überlauf mit gasdichter Feststoffschleuse wird das Katalysatorvolumen im Reaktor konstant gehalten. 100 Teile kontinuierlich ausgeschleuster Katalysator werden stündlich kontinuierlich in einem zweiten

Wirbelreaktor (Regenerierung) bei 440°C regeneriert. Als Regeneriergas wird stündlich ein Gemisch aus 200 000 Teilen Stickstoff und 4000 Teilen Sauerstoff zugeführt. Die mittlere Verweilzeit des Katalysators in der Regenerierzone beträgt 2 Stunden. Über einen Überlauf und eine gasdichte Schleuse wird das Katalysatorvolumen im zweiten Reaktor konstant gehalten. Der regenerierte Katalysator wird pneumatisch in einen Zwischenbehälter überführt. Aus diesem Zwischenbehälter wird der erste Wirbelreaktor mit Katalysator beschickt. Zum Ausgleich der Katalysatorverluste werden diskontinuierlich stündlich 5 Teile Katalysator dem Zwischenbehälter zugeführt. Die Ausbeute entspricht der von Beispiel 3. Auch nach 600 Stunden wird keine Veränderung im Katalysatorverhalten und in der Ausbeute festgestellt.

**Patentansprüche:**

1. Verfahren zur Herstellung von Imidazolen der Formel

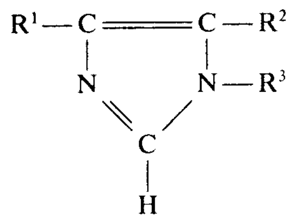

(I)

worin $R^1$, $R^2$ und $R^3$ gleich oder verschieden sein können und jeweils einen aliphatischen, cycloaliphatischen, araliphatischen oder aromatischen Rest oder ein Wasserstoffatom bedeuten, durch Umsetzung von N,N'-Diformyl-1,2-diaminen bei erhöhter Temperatur in Gegenwart von Metallkatalysatoren, dadurch gekennzeichnet, daß man N,N'-Diformyl-1,2-diamine der Formel

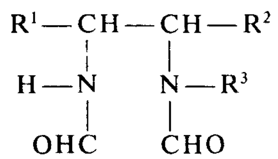

(II)

worin $R^1$, $R^2$ und $R^3$ die vorgenannte Bedeutung besitzen, bei einer Temperatur von 350 bis 500°C in Gegenwart von

a) Molybdänoxid und
b) Nickeloxid und/oder Kobaltoxid als Katalysatoren umsetzt.

2. Verfahren nach Anspruch 1, dadurch gekennzeichnet, daß man die Umsetzung in einem Wirbelbett durchführt, wobei ein Teil des Katalysators aus dem Reaktionsraum entnommen und der Regenerierung zugeführt wird und regenerierter Katalysator in den Reaktionsraum eingegeben wird.

**Claims**

1. A process for the production of an imidazole of the formula

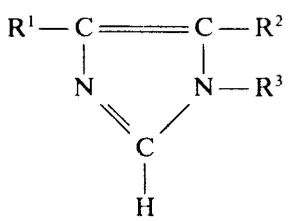

(I)

where $R^1$, $R^2$ and $R^3$ may be identical or different and each denotes an aliphatic, cycloaliphatic, araliphatic or aromatic radical or hydrogen, by reacting an N,N'-diformyl-1,2-diamine at elevated temperature in the presence of metal catalysts, wherein an N,N'-diformyl-1,2-diamine of the formula

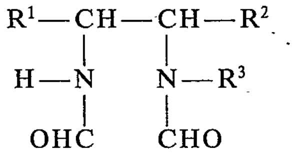

(II)

where $R^1$, $R^2$ and $R^3$ have the above meanings, is reacted at a temperature of from 350 to 500°C in the presence of

a) molybdenum oxid and
b) nickel oxide and/or cobalt oxide as catalysts.

2. A process as claimes in claim 1, wherein the reaction is carried out in a fluidized bed, part of the catalyst being removed from the reaction zone and supplied to the regeneration section, and regenerated catalyst being introduced into the reaction zone.

**Revendications**

1. Procédé pour la préparation d'imidazoles de formule

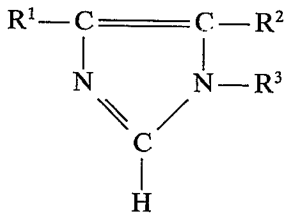

(I)

dans laquelle $R^1$, $R^2$ et $R^3$ peuvent être semblables ou différents et représentent chacun un radical aliphatique, cycloaliphatique, araliphatique ou aromatic ou un atome d'hydrogène, par transformation de N,N'-diformyl-1,2-diamines à température élevée en présence de catalyseurs métalliques, caractérisé en ce qu'on met à réagir des N,N'-diformyl-1,2-diamines de formule

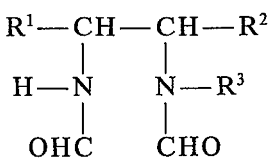

(II)

dans laquelle $R^1$, $R^2$ et $R^3$ ont les significations données ci-dessus, à une température de 350 à 500°C, en présence:

a) d'oxyde de molybdéne et
b) d'oxyde de nickel et/ou d'oxyde de cobalt en tant que catalyseurs.

2. Procédé selon la revendication 1, caractérisé en ce qu'on effectue la réaction dans un lit fluidisé, une partie du catalyseur étant prélevée dans la zone de réaction et dirigée vers la régénération, et du catalyseur régénéré étant réintroduit dans la zone de réaction.